(19) 

**Europäisches Patentamt**
**European Patent Office**
**Office européen des brevets**

(11) **EP 4 492 277 B1**

(12) **EUROPEAN PATENT SPECIFICATION**

(45) Date of publication and mention
of the grant of the patent:
**11.02.2026 Bulletin 2026/07**

(21) Application number: **23918048.2**

(22) Date of filing: **21.12.2023**

(51) International Patent Classification (IPC):
*G16C 60/00* (2019.01)     *G06F 30/20* (2020.01)
*G06F 17/15* (2006.01)     *G16C 20/10* (2019.01)
*G16C 20/30* (2019.01)

(52) Cooperative Patent Classification (CPC):
**G06F 30/20; G16C 20/10; G16C 20/30; G16C 60/00**

(86) International application number:
**PCT/CN2023/140454**

(87) International publication number:
**WO 2024/239633 (28.11.2024 Gazette 2024/48)**

(54) **METHOD FOR QUICKLY CALCULATING CARBON CONCENTRATION DISTRIBUTION DURING PULSE CARBURIZATION**

VERFAHREN ZUR SCHNELLEN BERECHNUNG DER KOHLENSTOFFKONZENTRATIONSVERTEILUNG WÄHREND DER IMPULSAUFKOHLUNG

PROCÉDÉ DE CALCUL RAPIDE DE DISTRIBUTION DE CONCENTRATION DE CARBONE PENDANT UNE CÉMENTATION PAR IMPULSION

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **25.05.2023 CN 202310608184**

(43) Date of publication of application:
**15.01.2025 Bulletin 2025/03**

(73) Proprietor: **Beijing Research Institute of Mechanical & Electrical Technology Co., Ltd. Cam Haidian District Beijing 100083 (CN)**

(72) Inventors:
• **MA, Jingbo**
  **Beijing 100083 (CN)**
• **CONG, Peiwu**
  **Beijing 100083 (CN)**
• **JIANG, Chao**
  **Beijing 100083 (CN)**
• **LU, Wenlin**
  **Beijing 100083 (CN)**
• **DU, Chunhui**
  **Beijing 100083 (CN)**

• **YAO, Jiawei**
  **Beijing 100083 (CN)**
• **YANG, Guangwen**
  **Beijing 100083 (CN)**
• **CHEN, Xuyang**
  **Beijing 100083 (CN)**
• **LIU, Junxiang**
  **Beijing 100083 (CN)**
• **HE, Longxiang**
  **Beijing 100083 (CN)**
• **XUE, Danruo**
  **Beijing 100083 (CN)**

(74) Representative: **Lorenz & Kollegen Lorenz & Kollegen Patentanwälte Partnerschaftgesellschaft mbB Alte Ulmer Straße 2 89522 Heidenheim (DE)**

(56) References cited:
**CN-A- 102 063 110     CN-A- 110 457 834**
**CN-A- 116 306 059     JP-A- 2008 208 403**
**US-A1- 2016 123 951     US-A1- 2016 123 951**

**(Cont. next page)**

- **GAO WEI MIN ET AL: "Integral Approach and Numerical Improvement to Calculate Carbon Concentration Profiles in Carburising", ADVANCED MATERIALS RESEARCH (DURNTEN-ZURICH, SWITZERLAND), vol. 264-265, 1 June 2011 (2011-06-01), pages 1494 - 1499, XP093273435, ISSN: 1662-8985, DOI: 10.4028/www.scientific.net/AMR.264-265.1494**
- **ZAJUSZ M ET AL: "Modeling of vacuum pulse carburizing of steel", SURFACE AND COATINGS TECHNOLOGY, ELSEVIER, NL, vol. 258, 19 August 2014 (2014-08-19), pages 646 - 651, XP029020221, ISSN: 0257-8972, DOI: 10.1016/J.SURFCOAT.2014.08.023**
- **MARIN FATIMA MATAMOROS ET AL: "Dynamic optimization of low-pressure carburizing furnaces", 2021 23RD INTERNATIONAL CONFERENCE ON PROCESS CONTROL (PC), IEEE, 1 June 2021 (2021-06-01), pages 72 - 77, XP033923852, DOI: 10.1109/PC52310.2021.9447496**

## Description

This patent application claims the benefit and priority of Chinese Patent Application No. CN202310608184.9 filed with the China National Intellectual Property Administration on May 25, 2023, and entitled "METHOD FOR RAPIDLY CALCULATING CARBON CONCENTRATION DISTRIBUTION IN PULSE CARBURIZING PROCESS".

## TECHNICAL FIELD

The present disclosure relates to the field of vacuum low-pressure carburizing, and in particular, to a method for calculating a carbon concentration distribution in a pulse carburizing process.

## BACKGROUND

Vacuum low-pressure carburizing originated from vacuum solid carburizing in the 1960s. In the 1970s, the vacuum solid carburizing had been gradually evolved into carburizing using a low-pressure gas and pulses, and vacuum carburizing dedicated equipment had emerged. In the 1990s, the concept of low-pressure carburizing had been clearly put forward, and it was experimentally demonstrated the advantages of acetylene as a carburizing medium in reducing carbon black production. After 2000, with the progress of research, a vacuum low-pressure carburizing process with acetylene as the carburizing medium and pulses as a working manner had been created. Along with worldwide problems such as environmental degradation and energy crisis, vacuum low-pressure carburizing, as a clean, efficient, and green thermal treatment technique, had received widespread attention.

Obtaining a carbon concentration distribution at a certain time in a carburizing process lays a foundation for describing and controlling a vacuum carburizing process. In atmosphere carburizing, a carbon concentration distribution in a carburizing process can be directly calculated by solving Fick law. Atmosphere carburizing also allows for use of an oxygen probe to measure a carbon potential in real time. Based on the above features, atmosphere carburizing enables real-time display of a carbon concentration variation in the carburizing process. However, this cannot be achieved in a vacuum carburizing process. Due to a vacuum environment, an oxygen probe cannot be used in the vacuum carburizing process and a real-time data feedback cannot be obtained. Meanwhile, vacuum carburizing involves an intensive carburizing-diffusion cyclic pulse process and is complex, and Fick law can only be solved by using a numerical calculation method. According to the numerical calculation method, an equation (e.g., Fick law) may be discretized in space and time by using a finite element method or a finite difference method, and then is solved to obtain a carbon concentration distribution at a certain time in a carburizing process. The accuracy of the numerical calculation method depends on a degree of discretization of the carburized layer in space and time. Therefore, the numerical calculation method often takes a long time for calculation and requires a lot of calculation resources, and cannot calculate a carbon concentration distribution directly and rapidly.

Prior art document US 2016/123951 A1 discloses the following content: a method of calculation of carbon concentration distribution in a member as a result of carburizing includes calculating n-dimensional carbon diffusion of the member, determining calculation region for which (n+1)-dimensional carbon diffusion of the member is calculated, determining a boundary condition of the (n+1)-dimensional calculation region, and calculating the (n+1)-dimensional carbon diffusion by applying the n-dimensional calculation result to the boundary condition of the (n+1)-dimensional calculation region. Further, software for calculating carbon concentration distribution in a member as a result of carburizing includes outputting a calculation result by calculating n-dimensional carbon diffusion of the member, and outputting a calculation result by calculating (n+1)-dimensional carbon diffusion of the member by applying the n-dimensional calculation result to a boundary condition of a calculation region for which the (n+1)-dimensional carbon diffusion of the member is calculated.

## SUMMARY

In order to solve the above technical problems, the present disclosure provides a method for calculating a gas flow rate in a pulse carburizing process that can provide a gas mass flow rate of a carburizing process, realize high accuracy control of a vacuum low-pressure carburizing process, and increase a carbon utilization ratio of a carburizing gas.

A method for rapidly calculating a carbon concentration distribution in a pulse carburizing process includes the following steps:

calculation of a carburized layer depth: calculating a distance of a position where a carbon concentration first reaches a matrix carbon concentration from a surface to a core part, from the surface when a carburizing stage ends;

segmentation of a carburized layer: segmenting the carburized layer into finite segments within the carburized layer depth;

curve fitting: fitting a carbon concentration distribution using a polynomial within each segment of the carburized layer; and

integral calculation of a carbon concentration: substituting a fitted total carbon concentration distribution into an integral equation of Green's function method to ascertain a carbon concentration distribution curve of next carburizing stage.

On the basis of the above solution, the segmentation of a carburized layer may include averagely setting discontinuity points in an equal division manner.

On the basis of the above solution, the curve fitting may include: within each segment of the carburized layer, using a one-variable polynomial containing n+1 terms, selecting n positions, and performing the curve fitting according to the positions and corresponding carbon concentrations by a Lagrangian interpolation method.

On the basis of the above solution, a piecewise function $M(z)$ describing a carbon concentration distribution of the carburized layer obtained by the curve fitting may be in the following form:

$$M(z) = \begin{cases} \sum_{i=0}^{3} a_{1i} z^i, 0 \le z < L_1 \\ \sum_{i=0}^{3} a_{2i} z^i, L_1 \le z < L_2 \\ \sum_{i=0}^{3} a_{3i} z^i, L_2 \le z < L_3 \\ \sum_{i=0}^{3} a_{4i} z^i, L_3 \le z < L_4 \\ C_b, L_4 \le z < +\infty \end{cases}$$

where $L_1$ to $L_4$ represent the discontinuity points; $C_b$ represents the matrix carbon concentration; $a_{ij}$ represents a coefficient of the piecewise function; $z$ represents the carburized layer depth; and $M(z)$ represents the carbon concentration distribution of the carburized layer.

On the basis of the above solution, the integral calculation of a carbon concentration may include calculating a field generated with different carbon potential point sources at any initial carbon concentration.

On the basis of the above solution, the integral calculation of a carbon concentration may include:

if next stage is a boost process, the Green's function is a Green's function corresponding to a third-kind boundary condition; and

if next stage is a diffusion process, the Green's function is a Green's function corresponding to a second-kind boundary condition and having a flow rate of zero.

On the basis of the above solution, the integral calculation of a carbon concentration may specifically include:

substituting the piecewise function describing a carbon concentration distribution of the carburized layer obtained by the curve fitting into the integral equation of Green's function method;

where if next stage is the diffusion process, the integral equation is as follows:

$$C_D(x,t) = \int_0^{+\infty} G_D(x,z,t) M(z) dz$$

where

$$G_D(x,z,t) = \frac{1}{\sqrt{4D\pi t}} \left\{ \exp\left[ -\frac{(x-z)^2}{4Dt} \right] + \exp\left[ -\frac{(x+z)^2}{4Dt} \right] \right\}$$

an integral is solved to obtain a carbon concentration distribution $C_D(x,t)$ of next stage;
if next stage is the boost process, the integral equation is as follows:

$$C_B(x,t) = C_D(x,t) + \int_0^{+\infty} G_F(x,z,t)M(z)dz + C_{Bd}(x,t)$$

where

$$C_{Bd}(x,t) = C_g \left\{ \text{erfc}\left( \frac{x}{2\sqrt{Dt}} \right) - \exp\left[ \left(B\sqrt{Dt}\right)^2 + Bx \right] \text{erfc}\left( B\sqrt{Dt} + \frac{x}{2\sqrt{Dt}} \right) \right\}$$

$$G_F(x,z,t) = -B \exp\left[ B(x+z) + \left(B\sqrt{Dt}\right)^2 \right] \text{erfc}\left\{ \frac{x+z}{2\sqrt{Dt}} + B\sqrt{Dt} \right\}$$

an integral is solved to obtain a carbon concentration distribution $C_B(x,t)$ of next stage;

On the basis of the above solution, the method may further include: with a carbon concentration distribution as an initial state, circularly performing the calculation of a carburized layer depth, the segmentation of a carburized layer, the curve fitting, and the integral calculation of a carbon concentration to obtain carbon concentration distributions of all carburizing processes.
On the basis of the above solution, a gas flow rate required in a carburizing process may be calculated according to the carbon concentration distribution; and

the calculation process may include:
calculating a surface carbon concentration $C_S = C_B(0,t)$ according to the carbon concentration distribution; and
calculating a gas mass flow rate according to the obtained $C_s$ and the following formula:

$$J = \frac{13}{12}\rho\beta A\left(C_g - C_S\right),$$

where $J$, $\rho$, $A$, $\beta$, and $C_g$ represent the gas mass flow rate, a density of a workpiece, an area of the workpiece, the surface transfer coefficient, and a carbon potential, respectively.

The present disclosure further provides a computer-readable storage medium, storing a computer program, where when executed by a processor, the computer program is caused to implement the method for rapidly calculating a carbon concentration distribution in a pulse carburizing process as described in the above technical solution.
The present disclosure has following beneficial effects:

1. The calculation method does not involve an iterative calculation process, can rapidly calculate a carbon concentration distribution at a certain time in the pulse carburizing process, distinguish between the boost process and the diffusion process based on a boundary condition, describe a vacuum low-pressure carburizing process, and present variations of a surface carbon concentration and a carburized depth in the vacuum low-pressure carburizing process over time.
2. The calculation method has high calculation efficiency, can be embedded in a computer such as an industrial personal computer with ordinary performance for use, may realize real-time display of a carbon concentration in a vacuum carburizing process, and can be used for industrial visual software. By the calculation method in the present disclosure, a vacuum low-pressure carburizing process may be designed. By the method in combination with an iterative calculation method (such as ascertaining a maximum and a minimum using a numerical calculation method), a time to reach a certain surface carbon concentration or a carburized layer depth (a carbon content is 0.35 wt%) is given, whereby a specific process is designed. By comparing carburizing times of different carburizing manners, a carburizing manner with a short process time may be selected, giving full play to efficient and green technical characteristics of vacuum low-pressure carburizing.

3. The method is also applicable to any combination of a finite number of intensive carburizing and diffusion processes, and can be promoted widespread. Meanwhile, the method may use a discrete carbon concentration distribution including a finite number of position-carbon concentration points and has intensive practicability.

The invention is set out in the appended set of claims.

**BRIEF DESCRIPTION OF THE DRAWINGS**

The present disclosure has the following drawings and tables:

FIG. 1 shows carbon concentration distributions at different times in a fifth carburizing process (a boost process) obtained by a calculation method in the present disclosure in Example 1;
FIG. 2 shows carbon concentration distributions at different times in a sixth carburizing process (a diffusion process) obtained by a calculation method in the present disclosure in Example 1;
FIG. 3 shows a relationship of a surface carbon concentration and a time in a carburizing process in Example 1;
FIG. 4 shows a relationship of a carburized layer depth and a time in a carburizing process in Example 1;
FIG. 5 shows carbon concentration distributions at different times in a first carburizing process (a boost process) obtained by a calculation method in the present disclosure in Example 2; and
FIG. 6 shows carbon concentration distributions at different times in a second carburizing process (a boost process) obtained by a calculation method in the present disclosure in Example 2; and

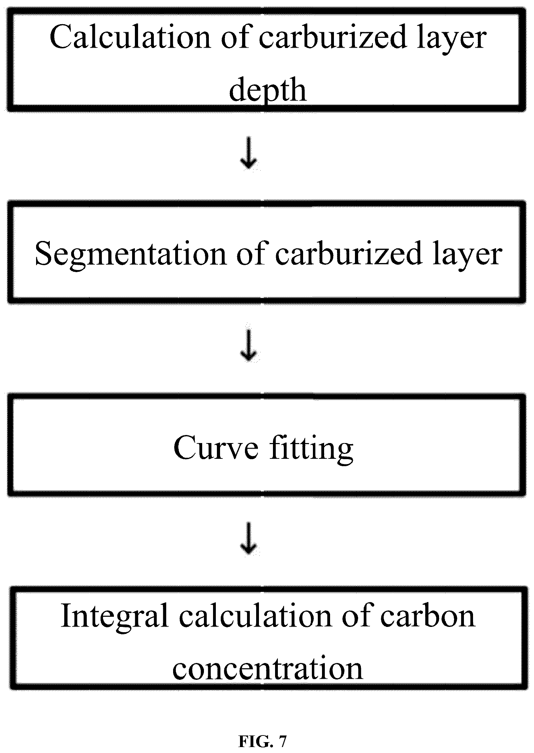

FIG. 7 is a block diagram showing steps of the present disclosure.

**DETAILED DESCRIPTION OF THE EMBODIMENTS**

The present disclosure will be further explained in conjunction with the drawings and tables as well as specific examples. It should be understood that these examples are intended to illustrate the present disclosure rather than limit the scope of the present disclosure.

A method for rapidly calculating a carbon concentration distribution in a pulse carburizing process includes calculation of a carburized layer depth, segmentation of a carburized layer, curve fitting, and integral calculation of a carbon concentration.

The calculation of a carburized layer depth refers to calculating a distance of a position where a carbon concentration first reaches a core part carbon concentration from a surface to a core part, from the surface when a carburizing stage ends.

The segmentation of a carburized layer refers to segmenting the carburized layer into finite segments within the carburized layer depth.

The curve fitting refers to fitting a carbon concentration distribution using a polynomial within each segment of the carburized layer.

The integral calculation of a carbon concentration refers to substituting a fitted total carbon concentration distribution into an integral equation of Green's function method to ascertain a carbon concentration distribution curve of next carburizing stage.

The In order to more clearly show the technical solutions provided by the present disclosure and the technical effects produced, the present disclosure will be described in detail below with specific examples.

Example 1:

A surface transfer coefficient of a workpiece is $4.48\times10^{-8}$ m/s; a diffusion coefficient is $1.49\times10^{-11}$ m²/s; a surface area of the workpiece is 1 m²; and a density of the workpiece is $7.8\times10^3$ kg/m³.

A time and a type of each process during a carburizing process are as shown in Table 1.

Table 1

| Process No. | Carburizing Process Type | Time, s |
|---|---|---|
| 1 | Boost process | 505 |
| 2 | Diffusion process | 178 |
| 3 | Boost process | 137 |
| 4 | Diffusion process | 243 |
| 5 | Boost process | 121 |
| 6 | Diffusion process | 302 |
| 7 | Boost process | 114 |
| 8 | Diffusion process | 359 |
| 9 | Boost process | 109 |
| 10 | Diffusion process | 415 |
| 11 | Boost process | 107 |
| 12 | Diffusion process | 470 |
| 13 | Boost process | 104 |
| 14 | Diffusion process | 1695 |

A matrix carbon concentration is 0.2 wt.%, and a carburizing gas is acetylene.

A method for calculating a carbon gas flow rate in a pulse carburizing process is carried out according to the following steps.

(1) An initial state is a non-carburized state of a workpiece, i.e., an internal carbon concentration of the workpiece is a matrix carbon concentration; and a carbon concentration distribution of an initial process is identical to that of atmosphere carburizing.

(2) Calculation of a carburized layer depth: using a numerical algorithm, a distance of a position where a carbon concentration first reaches a core part carbon concentration from a surface to a core part, from the surface when a carburizing stage ends is calculated as the carburized layer depth.

(3) Segmentation of a carburized layer: the carburized layer is equally divided into four segments.

(4) Curve fitting: within each segment of the carburized layer, a cubic one-variable polynomial containing 4 terms are used, positions which are 0, 0.3, 0.6, and 1 of a length of each segment are selected, and the curve fitting is performed according to the positions and corresponding carbon concentrations by a Lagrangian interpolation method.

(5) Integral calculation of a carbon concentration distribution: the integral calculation of a carbon concentration refers to calculating a field generated with different carbon potential point sources at any initial carbon concentration.

A piecewise function $M(z)$ describing a carbon concentration distribution of the carburized layer is obtained by the previous fitting process, and $M(z)$ is used to describe an initial carbon concentration distribution and is in the following form:

$$M(z) = \begin{cases} \sum_{i=0}^{3} a_{1i}z^i, & 0 \leq z < L_1 \\ \sum_{i=0}^{3} a_{2i}z^i, & L_1 \leq z < L_2 \\ \sum_{i=0}^{3} a_{3i}z^i, & L_2 \leq z < L_3 \\ \sum_{i=0}^{3} a_{4i}z^i, & L_3 \leq z < L_4 \\ C_b, & L_4 \leq z < +\infty \end{cases}$$

where $L_1$ to $L_4$ represent discontinuity points; $C_b$ represents the matrix carbon concentration, and $a_{ij}$ represents a

coefficient of the piecewise function, obtained by calculation in the previous step. $M(z)$ is substituted into an integral equation of Green's function method.

If next stage is a diffusion process, i.e., a point source is constantly zero, the integral equation of the Green's function method is as follows:

$$C_D(x,t) = \int_0^{+\infty} G_D(x,z,t) M(z) dz$$

where

$$G_D(x,z,t) = \frac{1}{\sqrt{4D\pi t}} \left\{ \exp\left[ -\frac{(x-z)^2}{4Dt} \right] + \exp\left[ -\frac{(x+z)^2}{4Dt} \right] \right\}$$

An integral is solved to obtain a carbon concentration distribution $C_D(x,t)$ of next process.

If next carburizing process is a boost process, the boost process has a point source where a carbon potential remains unchanged, and the integral equation of the Green's function method is as follows:

$$C_B(x,t) = C_D(x,t) + \int_0^{+\infty} G_F(x,z,t) M(z) dz + C_{Bd}(x,t)$$

where

$$C_{Bd}(x,t) = C_g \left\{ \text{erfc}\left( \frac{x}{2\sqrt{Dt}} \right) - \exp\left[ \left( B\sqrt{Dt} \right)^2 + Bx \right] \text{erfc}\left( B\sqrt{Dt} + \frac{x}{2\sqrt{Dt}} \right) \right\}$$

$$G_F(x,z,t) = -B \exp\left[ B(x+z) + \left( B\sqrt{Dt} \right)^2 \right] \text{erfc}\left\{ \frac{x+z}{2\sqrt{Dt}} + B\sqrt{Dt} \right\}$$

An integral is solved to obtain a carbon concentration distribution $C_D(x,t)$ of next process.

If the current carburizing process is a linear carbon potential rising process, i.e., a point source where a carbon potential linearly rises, equations are as follows:

$$G(x,z,t) = \frac{1}{\sqrt{4D\pi(t-\tau)}} \left\{ \exp\left[ -\frac{(x-z)^2}{4D(t-\tau)} \right] + \exp\left[ -\frac{(x+z)^2}{4D(t-\tau)} \right] \right\}$$

$$-B \exp\left[ B(x+z) + \left( B\sqrt{D(t-\tau)} \right)^2 \right] \text{erfc}\left\{ \frac{x+z}{2\sqrt{D(t-\tau)}} + B\sqrt{D(t-\tau)} \right\} \quad \text{(Formula 1)}$$

$$S(\tau) = \beta \left[ \left( C_g - C_b \right) \frac{\tau}{t_0} + C_b \right] \quad \text{(Formula 2)}$$

where $D$ represents a diffusion coefficient (m²/s);
$\beta$ represents a surface transfer coefficient (m/s);

$$B: \frac{\beta}{D};$$

$C_g$ represents a carbon potential (wt.%);
$C_b$ represents the matrix carbon concentration (wt.%); and
$t_0$ represents a time for the carbon potential to rise from the matrix carbon concentration $C_b$ to $C_g$.

An integral is solved so that the carbon concentration distribution $C(x,t)$ of the current carburizing process can be obtained. $\tau$ represents a carburizing time for integral calculation; and $z$ represents a carburized layer depth for integral calculation.

In addition, a process in which a surface carbon concentration remains unchanged is further included.

(6) For each carburizing process, the calculation processes of step (2) to step (5) are repeated to obtain carbon concentration distributions of all carburizing processes.

Result analysis:

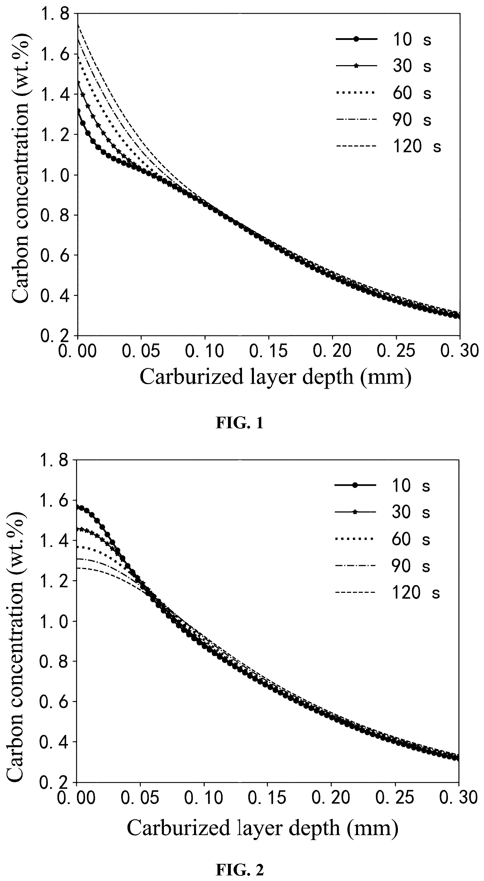

FIG. 1 shows carbon concentration distributions at different times in a fifth carburizing process (the boost process) obtained by the calculation method in the present disclosure. As can be seen from the figure, during the boost process, the carbon concentration within the carburized layer increases constantly with increasing carburizing time. The carbon concentration increases faster when getting closer to the surface.

FIG. 2 shows carbon concentration distributions at different times in a sixth carburizing process (the diffusion process) obtained by the calculation method in the present disclosure. As can be seen from the figure, during the diffusion process, the carbon concentration at a position closer to the surface decreases constantly with increasing carburizing time, while the carbon concentration at a position closer to the core part increases constantly. FIG. 1 and FIG. 2 indicate that the calculation method in the present disclosure can effectively calculate the carbon concentration distribution during the carburizing process. Under the condition of single core calculation at a primary frequency 1.60 GHz, the whole calculation process takes 12 seconds, which is far less than 10-20 minutes for existing finite element software, proving that the calculation method in the present disclosure realizes rapid calculation.

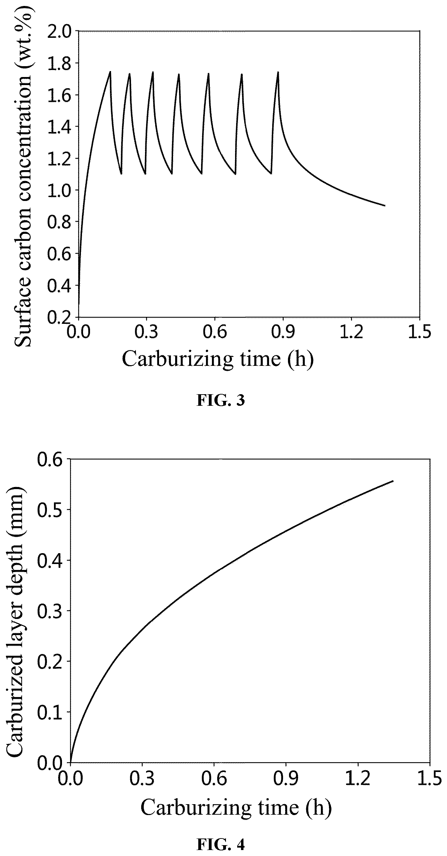

FIG. 3 shows a relationship of a surface carbon concentration and a time in a carburizing process. As can be seen from the figure, the surface carbon concentration increases in the boost process and decreases in the diffusion process. FIG. 4 shows relationship of a carburized layer depth and a time in a carburizing process. As can be seen from the figure, the carburized layer depth increases constantly with creasing time. FIG. 3 and FIG. 4 indicate that the calculation method in the present disclosure can give information such as a surface carbon concentration variation and a carburized layer depth variation in the carburizing process.

Example 2:

A surface transfer coefficient of a workpiece is $3.9 \times 10^{-8}$ m/s; a diffusion coefficient is $1.4 \times 10^{-11}$ m²/s; a surface area of the workpiece is 1 m²; and a density of the workpiece is $7.8 \times 10^3$ kg/m³.

Table 2

| Process No. | Carburizing Process Type | Time, s |
|---|---|---|
| 1 | Boost process | 100 |
| 2 | Boost process | 133 |
| 3 | Diffusion process | 1629 |
| 4 | Boost process | 222 |
| 5 | Diffusion process | 2414 |
| 6 | Boost process | 219 |
| 7 | Diffusion process | 799 |

A time and a type of each process during a carburizing process are as shown in Table 2, and an initial carbon concentration distribution is as shown in Table 3, with a carburizing gas: acetylene.

Table 3

| Position | Carbon concentration |
|---|---|
| 0 | 0.7 |
| 0.042 | 0.682 |
| 0.083 | 0.633 |
| 0.125 | 0.562 |
| 0.167 | 0.482 |
| 0.208 | 0.404 |
| 0.25 | 0.338 |
| 0.292 | 0.287 |
| 0.333 | 0.252 |
| 0.375 | 0.229 |
| 0.417 | 0.215 |
| 0.458 | 0.207 |
| 0.5 | 0.203 |

A method for calculating a carbon concentration distribution in a pulse carburizing process is carried out according to the following steps.

(1) an initial state is a carbon concentration distribution shown in Table 3.

(2) Calculation of a carburized layer depth: using a numerical algorithm, a distance of a position where a carbon concentration first reaches a core part carbon concentration from a surface to a core part, from the surface when a carburizing stage ends is calculated as the carburized layer depth. For a discrete carbon concentration distribution, the last point is directly used as the carburized layer depth. The carburized layer depth of the discrete carbon concentration distribution in this example is 0.5 mm.

(3) Segmentation of a carburized layer: the carburized layer is divided into four segments, which are 0-0.26 (a first segment), 0.26-0.49 (a second segment), 0.49-0.73 (a third segment), and 0.73-1 (a fourth segment) of the carburized layer depth. For the discrete carbon concentration distribution, four segments are divided in such a manner that 4 points are taken as one segment.

(4) Curve fitting: within each segment of the carburized layer, a cubic one-variable polynomial containing 4 terms are used, positions which are 0, 0.35, 0.70, and 1 of a length of each segment are selected, and the curve fitting is performed according to the positions and corresponding carbon concentrations by a Lagrangian interpolation method. For the discrete carbon concentration distribution, 4 points in each segment are directly used as data for curve fitting.

(5) Integral calculation of a carbon concentration distribution: a piecewise function $M(z)$ obtained in the previous process is in the following form:

$$M(z) = \begin{cases} \sum_{i=0}^{3} a_{1i} z^i, & 0 \leq z < L_1 \\[2ex] \sum_{i=0}^{3} a_{2i} z^i, & L_1 \leq z < L_2 \\[2ex] \sum_{i=0}^{3} a_{3i} z^i, & L_2 \leq z < L_3 \\[2ex] \sum_{i=0}^{3} a_{4i} z^i, & L_3 \leq z < L_4 \\[2ex] C_b, & L_4 \leq z < +\infty \end{cases}$$

where $L_1$ to $L_4$ represent discontinuity points; $C_b$ represents the matrix carbon concentration, and $a_{ij}$ represents a

coefficient of the piecewise function, obtained by calculation in the previous step. $M(z)$ is substituted into an integral equation of Green's function method. If next stage is a diffusion process, the equation is as follows:

$$C_D(x,t) = \int_0^{+\infty} G_D(x,z,t)M(z)dz$$

where

$$G_D(x,z,t) = \frac{1}{\sqrt{4D\pi t}}\left\{\exp\left[-\frac{(x-z)^2}{4Dt}\right] + \exp\left[-\frac{(x+z)^2}{4Dt}\right]\right\}$$

An integral is solved to obtain a carbon concentration distribution $C_D(x,t)$ of next process.

If the current carburizing process is a boost process, the equation is as follows:

$$C_B(x,t) = C_D(x,t) + \int_0^{+\infty} G_F(x,z,t)M(z)dz + C_{Bd}(x,t)$$

where

$$C_{Bd}(x,t) = C_g\left\{\text{erfc}\left(\frac{x}{2\sqrt{Dt}}\right) - \exp\left[\left(B\sqrt{Dt}\right)^2 + Bx\right]\text{erfc}\left(B\sqrt{Dt} + \frac{x}{2\sqrt{Dt}}\right)\right\}$$

$$G_F(x,z,t) = -B\exp\left[B(x+z) + \left(B\sqrt{Dt}\right)^2\right]\text{erfc}\left\{\frac{x+z}{2\sqrt{Dt}} + B\sqrt{Dt}\right\}$$

An integral is solved to obtain a carbon concentration distribution $C_B(x,t)$ of next process.

(6) For each carburizing process, the calculation processes of step (2) to step (5) are repeated to obtain carbon concentration distributions of all carburizing processes.

Result analysis:

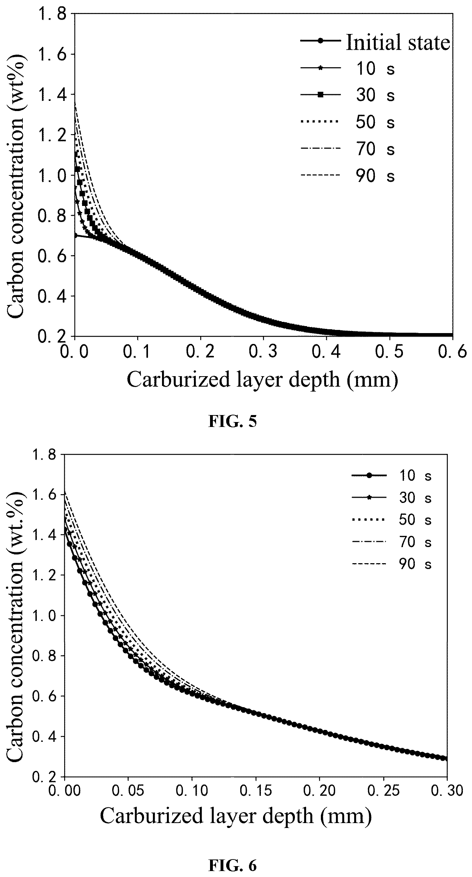

FIG. 5 shows carbon concentration distributions at different times in a first carburizing process (the boost process) obtained by the calculation method in the present disclosure. As can be seen from the figure, during the boost process, the carbon concentration within the carburized layer increases constantly with increasing carburizing time. FIG. 5 indicates the initial condition of the method is not limited to the carbon concentration distribution having an analytic equation, and the method is also applicable to a discrete carbon concentration distribution including a finite number of position-carbon concentration points and has strong practicability.

FIG. 6 shows carbon concentration distributions at different times in a second carburizing process (the boost process) obtained by the calculation method in the present disclosure. FIG. 5 and FIG. 6 indicate that the calculation method of the present disclosure is not limited to intensive carburizing-diffusion pulse cyclic process and is applicable to any combination of a finite number of intensive carburizing and diffusion processes, and can be promoted widespread. Example 2 shows that the method is also applicable when different workpiece parameters and curve fitting manners are adopted. Under the condition of single core calculation at a primary frequency 1.60 GHz, the whole calculation process takes 6 seconds (compared with Example 1, the steps are less), which is far less than 10-20 minutes for existing finite element software, proving that the calculation method in the present disclosure realizes rapid calculation.

The content not described in detail in the description belongs to the prior art well known to those skilled in the art.

**Claims**

1. A computer-implemented method for rapidly calculating a carbon concentration distribution in a pulse carburizing process, comprising:

   calculation of a carburized layer depth: calculating a distance of a position where a carbon concentration first reaches a matrix carbon concentration from a surface to a core part, from the surface when a carburizing stage ends;
   segmentation of a carburized layer: segmenting the carburized layer into finite segments within the carburized layer depth;
   curve fitting: fitting a carbon concentration distribution using a polynomial within each segment of the carburized layer; and
   integral calculation of a carbon concentration: substituting a fitted total carbon concentration distribution into an integral equation of Green's function method to ascertain a carbon concentration distribution curve of next carburizing stage;
   wherein the curve fitting comprises: within each segment of the carburized layer, using a one-variable polynomial containing n+1 terms, selecting n positions, and performing the curve fitting according to the positions and corresponding carbon concentrations by a Lagrangian interpolation method;
   the integral calculation of a carbon concentration comprises:

   substituting the function obtained by the curve fitting or a piecewise function $M(z)$ into the integral equation of Green's function method;
   wherein if next stage is a diffusion process, the integral equation is as follows:

   $$C_D(x,t) = \int_0^{+\infty} G_D(x,z,t)M(z)dz$$

   wherein

   $$G_D(x,z,t) = \frac{1}{\sqrt{4D\pi t}}\left\{\exp\left[-\frac{(x-z)^2}{4Dt}\right] + \exp\left[-\frac{(x+z)^2}{4Dt}\right]\right\}$$

   an integral is solved to obtain a carbon concentration distribution $C_D(x,t)$ of next stage; in the above formula, $x$ represents a carburized layer depth; $t$ represents a carburizing time; $z$ represents the carburized layer depth, which is an intermediate variable of the integral calculation; and $D$ represents a diffusion coefficient; and if next stage is a boost process, the integral equation is as follows:

   $$C_B(x,t) = C_D(x,t) + \int_0^{+\infty} G_F(x,z,t)M(z)dz + C_{Bd}(x,t)$$

   wherein

   $$C_{Bd}(x,t) = C_g\left\{\operatorname{erfc}\left(\frac{x}{2\sqrt{Dt}}\right) - \exp\left[\left(B\sqrt{Dt}\right)^2 + Bx\right]\operatorname{erfc}\left(B\sqrt{Dt} + \frac{x}{2\sqrt{Dt}}\right)\right\}$$

   $$G_F(x,z,t) = -B\exp\left[B(x+z) + \left(B\sqrt{Dt}\right)^2\right]\operatorname{erfc}\left\{\frac{x+z}{2\sqrt{Dt}} + B\sqrt{Dt}\right\}$$

   an integral is solved to obtain a carbon concentration distribution $C_B(x,t)$ of next stage; in the above formula, $\beta$ represents a surface transfer coefficient, $B = \dfrac{\beta}{D}$ ;

   wherein the computer-implemented method further comprises: with a carbon concentration distribution as an initial state, circularly performing the calculation of a carburized layer depth, the segmentation of a carburized layer, the curve fitting, and the integral calculation of a carbon concentration to obtain carbon concentration distributions of all carburizing processes; and

wherein the initial state is a non-carburized state of a workpiece, in which an internal carbon concentration of the workpiece is the matrix carbon concentration; and a carbon concentration distribution of an initial process is identical to that of atmosphere carburizing.

2. The computer-implemented method according to claim 1, wherein the segmentation of a carburized layer comprises averagely setting discontinuity points in an equal division manner.

3. The computer-implemented method according to claim 1, wherein the piecewise function $M(z)$ describing a carbon concentration distribution of the carburized layer obtained by the curve fitting is in the following form:

$$M(z) = \begin{cases} \sum_{i=0}^{3} a_{1i} z^i, 0 \le z < L_1 \\ \sum_{i=0}^{3} a_{2i} z^i, L_1 \le z < L_2 \\ \sum_{i=0}^{3} a_{3i} z^i, L_2 \le z < L_3 \\ \sum_{i=0}^{3} a_{4i} z^i, L_3 \le z < L_4 \\ C_b, L_4 \le z < +\infty \end{cases}$$

wherein $L_1$ to $L_4$ represent the discontinuity points; $C_b$ represents the matrix carbon concentration; $a_{ij}$ represents a coefficient of the piecewise function; $z$ represents the carburized layer depth; and $M(z)$ represents the carbon concentration distribution of the carburized layer.

4. The computer-implemented method according to claim 1, wherein the integral calculation of a carbon concentration comprises calculating a field generated with different carbon potential point sources at any initial carbon concentration.

5. The computer-implemented method according to claim 4, wherein the integral calculation of a carbon concentration comprises:

if next stage is the boost process, the Green's function is a Green's function corresponding to a third-kind boundary condition; and
if next stage is the diffusion process, the Green's function is a Green's function corresponding to a second-kind boundary condition and having a flow rate of zero.

6. The computer-implemented method according to claim 1, wherein a gas flow rate required in a carburizing process is calculated according to the carbon concentration distribution; and
the calculation process comprises:

calculating a surface carbon concentration $C_S = C_B(0,t)$ according to the carbon concentration distribution; and
calculating a gas mass flow rate according to the obtained $C_s$ and the following formula:

$$J = \frac{13}{12} \rho \beta A \left( C_g - C_S \right),$$

wherein $J$, $\rho$, $A$, $\beta$, and $C_g$ represent the gas mass flow rate, a density of the workpiece, an area of the workpiece, the surface transfer coefficient, and a carbon potential, respectively.

7. A computer-readable storage medium, storing a computer program, wherein when executed by a processor, the computer program is caused to implement the computer-implemented method for rapidly calculating a carbon concentration distribution in a pulse carburizing process according to any one of claims 1 to 6.

**Patentansprüche**

1. Computerimplementiertes Verfahren zur schnellen Berechnung einer Kohlenstoffkonzentrationsverteilung in einem Impulskohlenstoffisierungsprozess, umfassend:

Berechnung einer Aufkohlungslagentiefe: Berechnung eines Abstands einer Position, an der eine Kohlenstoffkonzentration zuerst eine Matrixkohlenstoffkonzentration erreicht, von einer Oberfläche zu einem Kernteil, von der Oberfläche aus, wenn eine Aufkohlungsstufe endet;
Segmentierung einer aufgekohlten Schicht: Segmentierung der aufgekohlten Schicht in endliche Segmente innerhalb der Tiefe der aufgekohlten Schicht;
Kurvenanpassung: Anpassen einer Kohlenstoffkonzentrationsverteilung unter Verwendung eines Polynoms innerhalb jedes Segments der aufgekohlten Schicht; und
Integralberechnung einer Kohlenstoffkonzentration: Einsetzen einer angepassten Gesamtkohlenstoffkonzentrationsverteilung in eine Integralgleichung der Green-Funktionsmethode, um eine Kohlenstoffkonzentrationsverteilungskurve der nächsten Aufkohlungsstufe zu ermitteln;
wobei die Kurvenanpassung umfasst: innerhalb jedes Segments der aufgekohlten Schicht unter Verwendung eines einvariablen Polynoms mit n+1 Termen, Auswählen von n Positionen und Durchführen der Kurvenanpassung gemäß den Positionen und den entsprechenden Kohlenstoffkonzentrationen durch ein Lagrange-Interpolationsverfahren;
die Integralberechnung einer Kohlenstoffkonzentration umfasst:

Ersetzen der durch die Kurvenanpassung erhaltenen Funktion oder einer stückweisen Funktion $M(z)$ in die Integralgleichung der Green-Funktionsmethode;
wobei, wenn die nächste Stufe ein Diffusionsprozess ist, die Integralgleichung wie folgt lautet:

$$C_D(x,t) = \int_0^{+\infty} G_D(x,z,t)M(z)dz$$

wobei

$$G_D(x,z,t) = \frac{1}{\sqrt{4D\pi t}}\left\{\exp\left[-\frac{(x-z)^2}{4Dt}\right] + \exp\left[-\frac{(x+z)^2}{4Dt}\right]\right\}$$

ein Integral gelöst wird, um eine Kohlenstoffkonzentrationsverteilung $C_D(x,t)$ der nächsten Stufe zu erhalten; in der obigen Formel $x$ für eine Aufkohlungslagentiefe steht; $t$ für eine Aufkohlungszeit steht; $z$ für die Aufkohlungslagentiefe steht, die eine Zwischenvariable der Integralberechnung ist; und $D$ für einen Diffusionskoeffizienten steht; und
wenn die nächste Stufe ein Boost-Prozess ist, lautet die Integralgleichung wie folgt:

$$C_B(x,t) = C_D(x,t) + \int_0^{+\infty} G_F(x,z,t)M(z)dz + C_{Bd}(x,t)$$

wobei

$$C_{Bd}(x,t) = C_g\left\{\mathrm{erfc}\left(\frac{x}{2\sqrt{Dt}}\right) - \exp\left[\left(B\sqrt{Dt}\right)^2 + Bx\right]\mathrm{erfc}\left(B\sqrt{Dt} + \frac{x}{2\sqrt{Dt}}\right)\right\}$$

$$G_F(x,z,t) = -B\exp\left[B(x+z)+\left(B\sqrt{Dt}\right)^2\right]\mathrm{erfc}\left\{\frac{x+z}{2\sqrt{Dt}}+B\sqrt{Dt}\right\}$$

ein Integral gelöst wird, um eine Kohlenstoffkonzentrationsverteilung $C_B(x,t)$ für die nächste Stufe zu erhalten; in der obigen Formel $\beta$ für einen Oberflächenübertragungskoeffizienten, $B=\frac{\beta}{D}$, steht;

wobei das computerimplementierte Verfahren ferner umfasst: mit einer Kohlenstoffkonzentrationsverteilung als Anfangszustand die zirkuläre Durchführung der Berechnung einer Aufkohlungslagentiefe, der Segmentierung einer Aufkohlungslage, der Kurvenanpassung und der Integralberechnung einer Kohlenstoffkonzentration, um Kohlenstoffkonzentrationsverteilungen aller Aufkohlungsprozesse zu erhalten; und wobei der Anfangszustand ein nicht aufgekohlter Zustand eines Werkstücks ist, in dem eine innere Kohlenstoffkonzentration des Werkstücks die Matrixkohlenstoffkonzentration ist; und eine Kohlenstoffkonzentrationsverteilung eines Anfangsprozesses identisch mit derjenigen der Atmosphärenaufkohlung ist.

2. Computerimplementiertes Verfahren nach Anspruch 1, wobei die Segmentierung einer aufgekohlten Schicht das durchschnittliche Setzen von Diskontinuitätspunkten in gleichmäßiger Teilung umfasst.

3. Computerimplementiertes Verfahren nach Anspruch 1, wobei die stückweise Funktion $M(z)$, die eine Kohlenstoffkonzentrationsverteilung der durch die Kurvenanpassung erhaltenen aufgekohlten Schicht beschreibt, die folgende Form hat:

$$M(z)=\begin{cases}\sum_{i=0}^{3}a_{1i}z^i, & 0\leq z<L_1\\[2mm]\sum_{i=0}^{3}a_{2i}z^i, & L_1\leq z<L_2\\[2mm]\sum_{i=0}^{3}a_{3i}z^i, & L_2\leq z<L_3\\[2mm]\sum_{i=0}^{3}a_{4i}z^i, & L_3\leq z<L_4\\[2mm]C_b, & L_4\leq z<+\infty\end{cases}$$

wobei $L_1$ bis $L_4$ die Diskontinuitätspunkte darstellen; $C_b$ die Matrixkohlenstoffkonzentration darstellt; $a_{ij}$ einen Koeffizienten der stückweisen Funktion darstellt; $z$ die Aufkohlungslagentiefe darstellt; und $M(z)$ die Kohlenstoffkonzentrationsverteilung der Aufkohlungslage darstellt.

4. Computerimplementiertes Verfahren nach Anspruch 1, wobei die Integralberechnung einer Kohlenstoffkonzentration die Berechnung eines Feldes umfasst, das mit verschiedenen Kohlenstoffpotenzial-Punktquellen bei einer beliebigen Anfangskohlenstoffkonzentration erzeugt wird.

5. Computerimplementiertes Verfahren nach Anspruch 4, wobei die Integralberechnung einer Kohlenstoffkonzentration umfasst:

wenn die nächste Stufe der Boost-Prozess ist, die Green-Funktion eine Green-Funktion ist, die einer Randbedingung dritter Art entspricht; und
wenn die nächste Stufe der Diffusionsprozess ist, ist die Green-Funktion eine Green-Funktion, die einer Randbedingung zweiter Art entspricht und eine Strömungsgeschwindigkeit von Null aufweist.

6. Computerimplementiertes Verfahren nach Anspruch 1, wobei eine in einem Aufkohlungsprozess erforderliche Gasströmungsrate gemäß der Kohlenstoffkonzentrationsverteilung berechnet wird; und
der Berechnungsprozess umfasst:

Berechnen einer Oberflächenkohlenstoffkonzentration $C_S = C_B(0,t)$ gemäß der Kohlenstoffkonzentrationsverteilung; und

Berechnen einer Gasmassendurchflussrate gemäß der erhaltenen $C_s$ und der folgenden Formel:

$$J = \frac{13}{12} \rho \beta A \left( C_g - C_S \right),$$

wobei $J$, $\rho$, $A$, $\beta$ und $C_g$ jeweils den Gasmassendurchfluss, die Dichte des Werkstücks, die Fläche des Werkstücks, den Oberflächenübertragungskoeffizienten und das Kohlenstoffpotenzial darstellen.

7. Computerlesbares Speichermedium, auf dem ein Computerprogramm gespeichert ist, wobei das Computerprogramm, wenn es von einem Prozessor ausgeführt wird, dazu veranlasst wird, das computerimplementierte Verfahren zur schnellen Berechnung einer Kohlenstoffkonzentrationsverteilung in einem Impulskohlenstoffisierungsprozess nach einem der Ansprüche 1 bis 6 zu implementieren.

**Revendications**

1. Procédé mis en œuvre par ordinateur pour calculer rapidement une distribution de concentration en carbone dans un processus de cémentation par impulsions, comprenant :

calcul d'une profondeur de couche cémentée : calculer une distance entre une position où la concentration en carbone atteint pour la première fois une concentration en carbone matricielle depuis une surface jusqu'à une partie centrale, à partir de la surface lorsque l'étape de cémentation se termine ;
segmentation d'une couche cémentée : la segmentation de la couche cémentée en segments finis dans la profondeur de la couche cémentée ;
ajustement de courbe : l'ajustement d'une distribution de la concentration en carbone à l'aide d'un polynôme dans chaque segment de la couche cémentée ; et
calcul intégral d'une concentration en carbone : substitution d'une distribution totale ajustée de la concentration en carbone dans une équation intégrale de la méthode de la fonction de Green afin de déterminer une courbe de distribution de la concentration en carbone de l'étape de cémentation suivante ;
dans lequel l'ajustement de courbe comprend : dans chaque segment de la couche cémentée, l'utilisation d'un polynôme à une variable contenant n+1 termes, la sélection de n positions et la réalisation de l'ajustement de courbe en fonction des positions et des concentrations de carbone correspondantes par une méthode d'interpolation lagrangienne ;
le calcul intégral d'une concentration en carbone comprend :

la substitution de la fonction obtenue par l'ajustement de courbe ou d'une fonction par morceaux $M(z)$ dans l'équation intégrale de la méthode de la fonction de Green ;
dans lequel, si l'étape suivante est un processus de diffusion, l'équation intégrale est la suivante :

$$C_D(x,t) = \int_0^{+\infty} G_D(x,z,t) M(z) dz$$

où

$$G_D(x,z,t) = \frac{1}{\sqrt{4D\pi t}} \left\{ \exp\left[ -\frac{(x-z)^2}{4Dt} \right] + \exp\left[ -\frac{(x+z)^2}{4Dt} \right] \right\}$$

une intégrale est résolue pour obtenir une distribution de la concentration en carbone $C_D(x,t)$ de l'étape suivante ; dans la formule ci-dessus, $x$ représente la profondeur de la couche cémentée ; $t$ représente le temps de cémentation ; $z$ représente la profondeur de la couche cémentée, qui est une variable intermédiaire du calcul intégral ; et $D$ représente un coefficient de diffusion ; et
si l'étape suivante est un processus d'accélération, l'équation intégrale est la suivante :

$$C_B(x,t) = C_D(x,t) + \int_0^{+\infty} G_F(x,z,t)M(z)dz + C_{Bd}(x,t)$$

où

$$C_{Bd}(x,t) = C_g \left\{ \operatorname{erfc}\left(\frac{x}{2\sqrt{Dt}}\right) - \exp\left[\left(B\sqrt{Dt}\right)^2 + Bx\right]\operatorname{erfc}\left(B\sqrt{Dt} + \frac{x}{2\sqrt{Dt}}\right)\right\}$$

$$G_F(x,z,t) = -B\exp\left[B(x+z) + \left(B\sqrt{Dt}\right)^2\right]\operatorname{erfc}\left\{\frac{x+z}{2\sqrt{Dt}} + B\sqrt{Dt}\right\}$$

un intégral est résolu pour obtenir une distribution de la concentration en carbone $C_B(x,t)$ de l'étape suivante ;

dans la formule ci-dessus, $\beta$ représente un coefficient de transfert de surface, $B = \frac{\beta}{D}$ ;

dans lequel le procédé mis en œuvre par ordinateur comprend en outre : avec une distribution de la concentration en carbone comme état initial, effectuer de manière circulaire le calcul d'une profondeur de couche cémentée, la segmentation d'une couche cémentée, l'ajustement de courbe et le calcul intégral d'une concentration en carbone afin d'obtenir des distributions de la concentration en carbone de tous les processus de cémentation ; et

dans lequel l'état initial est un état non cémenté d'une pièce à usiner, dans lequel une concentration interne en carbone de la pièce à usiner est la concentration en carbone de la matrice; et une distribution de la concentration en carbone d'un processus initial est identique à celle de la cémentation atmosphérique.

2. Procédé mis en œuvre par ordinateur selon la revendication 1, dans lequel la segmentation d'une couche cémentée comprend la définition moyenne de points de discontinuité de manière à les répartir de manière égale.

3. Procédé mis en œuvre par ordinateur selon la revendication 1, dans lequel $M(z)$ de la fonction par morceaux décrivant une distribution de la concentration en carbone de la couche cémentée obtenue par l'ajustement de courbe est sous la forme suivante :

$$M(z) = \begin{cases} \sum_{i=0}^{3} a_{1i}z^i, & 0 \leq z < L_1 \\[2mm] \sum_{i=0}^{3} a_{2i}z^i, & L_1 \leq z < L_2 \\[2mm] \sum_{i=0}^{3} a_{3i}z^i, & L_2 \leq z < L_3 \\[2mm] \sum_{i=0}^{3} a_{4i}z^i, & L_3 \leq z < L_4 \\[2mm] C_b, & L_4 \leq z < +\infty \end{cases}$$

où les points $L_1$ à $L_4$ représentent les points de discontinuité ; $C_b$ représente la concentration en carbone de la matrice ; $a_{ij}$ représente un coefficient de la fonction par morceaux ; $z$ représente la profondeur de la couche cémentée ; et $M(z)$ représente la distribution de la concentration en carbone de la couche cémentée.

4. Procédé mis en œuvre par ordinateur selon la revendication 1, dans lequel le calcul intégral d'une concentration en carbone comprend le calcul d'un champ généré avec différentes sources ponctuelles de potentiel de carbone à n'importe quelle concentration initiale en carbone.

**5.** Procédé mis en œuvre par ordinateur selon la revendication 4, dans lequel le calcul intégral d'une concentration en carbone comprend :

si l'étape suivante est le processus de suralimentation, la fonction de Green est une fonction de Green correspondant à une condition limite de troisième type ; et
si l'étape suivante est le processus de diffusion, la fonction de Green est une fonction de Green correspondant à une condition limite de deuxième type et ayant un débit nul.

**6.** Procédé mis en œuvre par ordinateur selon la revendication 1, dans lequel un débit de gaz requis dans un processus de cémentation est calculé en fonction de la distribution de la concentration en carbone ; et
le processus de calcul comprend :

le calcul d'une concentration de carbone en surface $C_S = C_B(0,t)$ en fonction de la distribution de la concentration en carbone ; et
le calcul d'un débit massique de gaz en fonction de la $C_s$ obtenue et de la formule suivante:

$$J = \frac{13}{12} \rho \beta A \left( C_g - C_S \right),$$

où $J$, $\rho$, $A$, $\beta$ et $C_g$ représentent respectivement le débit massique de gaz, la densité de la pièce, la surface de la pièce, le coefficient de transfert de surface et le potentiel de carbone.

**7.** Support de stockage lisible par ordinateur, stockant un programme informatique, dans lequel, lorsqu'il est exécuté par un processeur, le programme informatique est amené à mettre en œuvre le procédé mis en œuvre par ordinateur pour calculer rapidement une distribution de concentration en carbone dans un procédé de cémentation par impulsions selon l'une quelconque des revendications 1 à 6.

**FIG. 1**

**FIG. 2**

**FIG. 3**

**FIG. 4**

**FIG. 5**

**FIG. 6**

Calculation of carburized layer depth

↓

Segmentation of carburized layer

↓

Curve fitting

↓

Integral calculation of carbon concentration

FIG. 7

**EP 4 492 277 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202310608184

- US 2016123951 A1